# EUROPÄISCHE PATENTANMELDUNG

(11) **EP 4 620 403 A1**
(43) Veröffentlichungstag der Anmeldung: **24.09.2025**
(21) Anmeldenummer: 25158668.1
(22) Anmeldetag: 18.02.2025
(51) Int. Cl.: A61B 8/04, A61B 8/06, A61B 8/08, A61B 8/00

(54) **AUFSATZ FÜR EINE ULTRASCHALLSONDE**

(30) Priorität: 22.03.2024 CH 3122024
(71) Anmelder: Compremium AG, 3074 Muri b. Bern (CH)
(72) Erfinder: Baumann, Ulrich, 3110 Münsigen (CH); Frei, Peter Nuot, 4657 Dulliken (CH); Roth, Patrick, 3400 Burgdorf (CH); Milani, Federico, 3014 Bern (CH); Baumann, Vincent Boris, 3073 Gümligen (CH); Reinmann, Andreas, 4534 Flumenthal (CH)
(74) Vertreter: Keller Schneider Patent- und Markenanwälte AG

(57) **Zusammenfassung**

Ein Aufsatz (20) für eine Ultraschallsonde (10) umfasst einen Grundkörper (21) mit einer Aufnahme (25) für einen distalen Endbereich (12) der Ultraschallsonde (10), eine distal am Grundkörper (21) angeordnete I<ontal<tfläche, die durch eine ultraschalltransparente Membran (22) gebildet ist, und einen Drucksensor zur Messung eines auf die I<ontal<tfläche wirkenden Anpressdrucks. Dabei sind die Aufnahme (25) für die Ultraschallsonde und die I<ontal<tfläche derart angeordnet, dass ein Messstrahl (15) der Ultraschallsonde (10) die ultraschalltransparente Membran (22) durchdringt und dass zwischen dem Messstrahl (15) und einer Hauptfläche der I<ontal<tfläche ein Winkel von 35-80°, insbesondere 45-75°, gebildet ist. Der Aufsatz (20) ermöglicht eine präzise Bestimmung der Flussgeschwindigkeit in einem Blutgefäss (3) und eine vorteilhafte Handhabung zum Aufbringen einer äusseren Andruckkraft.

## Beschreibung

### Technisches Gebiet

Die Erfindung betrifft einen Aufsatz für eine Ultraschallsonde, umfassend einen Grundkörper mit einer Aufnahme für einen distalen Endbereich der Ultraschallsonde, eine distal am Grundkörper angeordnete I<ontal<tfläche, die durch eine ultraschalltransparente Membran gebildet ist, und einen Drucksensor zur Messung eines auf die I<ontal<tfläche wirkenden Anpressdrucks. Die Erfindung betrifft weiter eine Ultraschallsondenanordnung mit einem solchen Aufsatz und ein Verfahren zur Messung eines Innendrucks in einem Blutgefäss mit einer Ultraschallsondenanordnung.

### Stand der Technik

Aufsätze für Ultraschallsonden, die eine Druckmessung ermöglichen, sind an sich bekannt. Sie werden beispielsweise zur Untersuchung von Logen im Zusammenhang mit einem möglichen I<ompartmentsyndrom vorgeschlagen.

So zeigt die WO 2019/106535 A1 (V. Baumann) eine Messeinheit, zu der eine Druckmessvorrichtung und eine Ultraschallmesseinheit verbunden sind. Zur Kopplung der Druckmessvorrichtung mit der Ultraschallmesseinheit ist ein L-förmiger Adapter vorgesehen, der als Verbindungsstück zum Ultraschallkopf dient und die Position der Druckmessvorrichtung vor dem Ultraschallkopf sichert, so dass der zwischen Messeinheit und I<örperoberfläche auszuübende Druck auf die Druckmessvorrichtung übertragen werden kann. Das Drucl<messsystem umfasst einen Foliensensor.

Der Blutdruck lässt sich nicht-invasiv u. a. dadurch bestimmen, dass Blutgefässe durch einen externen Anpressdruck okkludiert werden und der Blutdruck anhand des Okklusionsdrucks bestimmt wird. Die Okklusion kann auf verschiedene Weise erfasst werden. Der Goldstandard wird dabei durch die Methode nach Riva-Rocci gebildet, bei der eine Druckmanschette am Oberarm, ungefähr auf Herzhöhe angelegt wird. Die Druckmanschette lässt sich aufpumpen, und sie ist mit einem Druckmesser verstehen über die der Anpressdruck erfasst werden kann. Die Druckmanschette wird aufgepumpt, bis der Blutfluss in der Arteria Brachialis sicher unterbunden ist. Danach wird der Druck über ein Stellventil langsam abgelassen. Der Nutzer erfasst mit Hilfe eines Stethoskops (z. B. in der Armbeuge) Flussgeräusche. Anhand dieser kann er erkennen, wann der Anpressdruck dem systolischen (Einsetzen des Blutflusses) bzw. dem diastolischen Blutdruck (laminare Strömung) entspricht. Es sind Geräte verfügbar, die im Wesentlichen dieses Verfahren automatisch durchführen können.

Das Verfahren ist aufgrund der benötigten Manschette recht aufwendig in der Durchführung. Die korrekte Erkennung der Flussgeräusche ist zudem anspruchsvoll und kann zu systematischen Unterschieden bei der Blutdruckbestimmung durch unterschiedliche Nutzer oder Blutdrucl<messgeräte führen.

Ebenfalls bekannt sind Handgeräte zur Blutdruckmessung, bei der Blutgefässe ohne Manschette durch einen externen Anpressdruck des Handgeräts okkludiert werden und der Blutdruck anhand des Okklusionsdrucks bestimmt wird. So beschreibt die US 2015/0374249 A1 (Léman Micro Devices) Handgeräte zur Gewinnung medizinischer Daten, die als Stand-alone-Geräte ausgebildet oder in lokale Endgeräte (Smartphones, Tablets etc.) integriert sein können. Die Geräte umfassen Okklusionsmittel, mit denen auf einen Körperteil eingewirkt werden kann, um ein Blutgefäss zu okkludieren; sie umfassen weiter Mittel zur Erkennung des Blutflusses in diesem Blutgefäss sowie einen Drucksensor zur Messung der Anpresskraft. Die Erkennung des Blutflusses kann mittels Doppler-Ultraschall erfolgen. Der Drucksensor kann in einer Flüssigkeit eingebettet sein, wobei die Flüssigkeit hinter einer Membran zurückgehalten wird, die die I<ontal<tfläche der Okklusionsmittel bildet.

Das Gerät ist zur Blutdruckmessung am Finger bestimmt. Es ist fraglich, welche Präzision damit erreichbar ist. Die modulare Druckmessvorrichtung der WO 2019/106535 A1 ist nicht auf eine Blutdruckmessung ausgerichtet.

### Darstellung der Erfindung

Aufgabe der Erfindung ist es, einen dem eingangs genannten technischen Gebiet zugehörenden Aufsatz für eine Ultraschallsonde zu schaffen, welcher eine einfache und zuverlässige Blutdruckmessung ermöglicht.

Die Lösung der Aufgabe ist durch die Merkmale des Anspruchs 1 definiert. Gemäss der Erfindung sind beim Aufsatz die Aufnahme für die Ultraschallsonde und die I<ontal<tfläche derart angeordnet, dass ein Messstrahl der Ultraschallsonde die ultraschalltransparente Membran durchdringt und dass zwischen dem Messstrahl und einer Hauptfläche der I<ontal<tfläche ein Winkel von 35-80°, insbesondere 45-75°, gebildet ist.

Die I<ontal<tfläche ist insbesondere konvex und mittels Krafteinwirkung verformbar sowie rotationssymmetrisch. In diesem Fall ist die Hauptfläche der I<ontal<tfläche als Ebene definiert, die bei fehlender Krafteinwirkung tangential am Zentrum der I<ontal<tfläche anliegt. Sie liegt senkrecht zur Symmetrieachse der I<ontal<tfläche und verschiebt sich bei einer homogenen Krafteinwirkung entlang der Symmetrieachse lediglich in Richtung der Symmetrieachse, so dass der erwähnte Winkel unverändert bleibt.

Ein Winkel im genannten Bereich ermöglicht insbesondere eine zuverlässige Erfassung von Flussgeschwindigkeiten in untersuchten Gefässen, z. B. Blutgefässen, besonders mittels Doppler-Ultraschall. Der Winkel beträgt insbesondere ca. 60-70°.

Eine erfindungsgemässe Ultraschallsondenanordnung umfasst entsprechend eine Ultraschallsonde und einen erfindungsgemässen Aufsatz.

Der Winkel zwischen Messstrahl und Hauptausdehnung des Blutgefässes ermöglicht eine präzise Bestimmung der Flussgeschwindigkeit anhand des Doppler-Echos. Die Geometrie des Aufsatzes, mit dem Winkel zwischen I<ontal<tfläche und Messstrahl, ermöglicht eine Untersuchung mit angewinkeltem Messstrahl, bei der die I<ontal<tfläche parallel zur I<örperoberfläche verläuft. Dadurch ergibt sich eine intuitive Handhabung und es wird insbesondere beim Aufbringen einer äusseren Andruckkraft senkrecht zur Tangentialebene am Einwirl<punl<t verhindert, dass die Ultraschallsondenanordnung auf der Haut abrutscht und sich der Messort verschiebt, was zu ungenauen oder gar unbrauchbaren Messungen führt.

Bevorzugt ist zwischen der ultraschalltransparenten Membran und dem Grundkörper ein Aufnahmeraum für eine ultraschalltransparente Flüssigkeit ausgebildet, und der Drucksensor ist zur Messung eines Drucks in der Flüssigkeit ausgebildet. Der Aufnahmeraum wird - wie die ultraschalltransparente Membran - vom Messstrahl durchdrungen. Die ultraschalltransparente Flüssigkeit kann unterschiedliche Viskositäten aufweisen. Es kann sich dabei beispielsweise um eine wässrige Lösung, ein Öl oder ein gelartiges Produkt handeln.

Der flüssigkeitsgefüllte Aufnahmeraum definiert die Form der Membran und ermöglicht eine intuitive und gut kontrollierbare Handhabung der Ultraschallsondenanordnung, insbesondere was die Ausübung von äusserem Druck auf die I<örperoberfläche anbelangt. Die Druckmessung in der Flüssigkeit kann durch einen Drucksensor erfolgen, der unmittelbar in der Flüssigkeit oder am Rand des Aufnahmeraums angeordnet ist oder der über eine Leitung kommunizierend mit dem Aufnahmeraum verbunden ist. Es sind verschiedenartige Drucksensoren einsetzbar, z. B. piezoresistive Sensoren.

Bei einer bevorzugten Ausführungsform der Erfindung hat die ultraschalltransparente Membran eine kreisförmige Grundfläche, und die Aufnahme ist derart aussermittig am Grundkörper angeordnet, dass der Messstrahl die ultraschalltransparente Membran in einem zentralen Bereich durchdringt, der einer Kreisfläche mit einem Aussenradius vom halben Aussenradius der kreisförmigen Grundfläche entspricht.

Als Messstrahl wird jeweils eine geometrische gerade Linie verstanden, die das Zentrum des ausgesandten Ultraschallstrahls repräsentiert. Diese Linie schneidet den zentralen Bereich der Membran, wobei Anteile der ausgesandten Ultraschallstrahlung durch den Bereich der Membran ausserhalb des zentralen Bereichs hindurchgehen können. Zumindest ein Teil der reflektierten Ultraschallstrahlung wird durch die Membran, im zentralen und/oder äusseren Bereich, auf den Ultraschall-Transducer zurückgeworfen.

Die Geometrie des Aufsatzes ist insbesondere so gewählt, dass die zentrale Achse der Membran (senkrecht zur Hauptfläche der I<ontal<tfläche) den Messstrahl in einem Abstand entlang der zentralen Membranachse von 0-20 mm, insbesondere 2-12 mm, schneidet. Der Anpressdruck wirkt so - bei gängigen Tiefen von zu untersuchenden Blutgefässen von einigen mm bis maximal 2 cm - im Wesentlichen dort, wo die Ultraschallmessungen durchgeführt werden.

Bevorzugt ist die Aufnahme konisch ausgebildet. Ein entsprechend konischer distaler Endbereich der Ultraschallsonde kann so einfach und stabil aufgenommen werden. Durch die Wahl einer Innenoberfläche der Aufnahme mit geeigneten Reibungskoeffizienten ergibt sich eine durch I<raftschluss ausreichend gesicherte Befestigung des Aufsatzes auf der Sonde. Stattdessen oder zusätzlich können Haltemittel zwischen Aufsatz und Sonde vorhanden sein.

Bei einer ersten Gruppe von Ausführungsformen handelt es sich bei der Ultraschallsonde um eine Einzelstrahl-Ultraschallsonde. Dadurch ergibt sich eine besonders einfache und kosteneffiziente Ultraschallsondenanordnung. Es hat sich gezeigt, dass insbesondere der Blutfluss gut mit Einzelstrahl-Doppler-Messungen (A-Mode) überwacht werden kann. Eine Bildgebung ist in diesem Fall nicht zwingend notwendig. Auch dies vereinfacht das Gesamtsystem und dessen Handhabung.

Bei einer zweiten Gruppe von Ausführungsformen ist die Ultraschallsonde zum Generieren mehrerer Messstrahlen entlang einer Linie ausgebildet. Sie umfasst somit ein Ultraschall-Array.

Mit einer Ultraschallsondenanordnung, die eine solche Ultraschallsonde mit mehreren Messstrahlen umfasst, kann anhand der mehreren Messstrahlen eine seitliche Abweichung zwischen Ultraschallsonde und Blutgefäss erkannt und beim Erkennen der Okklusion berücksichtigt werden. So kann auch bei unbeabsichtigten Verschiebungen der Sondenanordnung gegenüber dem Körper des Patienten oder bei einer Dislokation des untersuchten Blutgefässes aufgrund der äusseren Kraft die Erfassung des Blutgefässes sichergestellt werden.

Das Array wird in diesem Fall insbesondere im Sinn einer Kombination mehrerer Einzelstrahlen betrieben. In entsprechenden Vorrichtungen umfasst das Array beispielsweise 3-9 Transducer. Ein mittlerer Transducer kann als eigentliche Messsonde betrieben werden, während die seitlichen Transducer die Abweichung erfassen und bei Bedarf eine Korrektur der Messung ermöglichen. Die Abweichung wird beispielsweise erkannt, wenn die stärkste Dopplerl<omponente, entsprechend dem Blutfluss im Blutgefäss, nicht mehr mittig, sondern seitlich versetzt erkannt wird. In diesem Fall kann die Zuordnung des Messstrahls zum entsprechenden seitlichen Transducer erfolgen, oder der Nutzer wird aufgefordert, die Sondenanordnung wieder zu zentrieren.

In anderen Vorrichtungen wird das Array zur Bildgebung eingesetzt und umfasst in an sich bekannter Weise z. B. 128-512 Transducer.

Ein erfindungsgemässes Verfahren zur Messung eines Innendrucks in einem Blutgefäss mit einer Ultraschallsondenanordnung umfasst folgende Schritte:
a) Aufsetzen der Ultraschallsondenanordnung auf die I<örperoberfläche eines Patienten, an einem Aufsetzort;
b) Komprimieren eines Blutgefässes unterhalb des Aufsetzorts durch Erhöhung des Anpressdrucks einer I<ontal<tfläche der Ultraschallsondenanordnung auf die I<örperoberfläche;
c) Erkennen einer Okklusion des Blutgefässes anhand von Ultraschallsignalen einer Ultraschallsonde der Ultraschallsondenanordnung, wobei der zu messende Innendruck aus dem mittels eines Drucksensors der Ultraschallsondenanordnung gemessenen Anpressdruck der Ultraschallsondenanordnung bei einsetzender Okklusion bestimmt wird.

Das Verfahren wird insbesondere mit einer erfindungsgemässen Ultraschallsondenanordnung mit Ultraschallsonde und Aufsatz durchgeführt. In diesem Fall wird die distal am Grundkörper des Aufsatzes angeordnete I<ontal<tfläche auf den Aufsetzort aufgesetzt und wirkt zur Erhöhung des Anpressdrucks auf die I<örperoberfläche ein.

Das erfindungsgemässe Verfahren ist aber auch mit einer Vorrichtung durchführbar, bei der die I<ontal<tfläche und der Drucksensor mit der eigentlichen Ultraschallsonde in eine Einheit integriert sind.

Der zu messende Innendruck kann im einfachsten Fall, bei hoher Elastizität des Blutgefässes, mit dem Anpressdruck bei einsetzender Okklusion gleichgesetzt werden.

Bevorzugt wird ein Blutfluss im Blutgefäss anhand von Ultraschallsignalen der Ultraschallsonde überwacht, wobei die Okklusion anhand eines ausbleibenden Blutflusses erkannt wird.

Mit Vorteil wird der Blutfluss anhand von Doppler-Ultraschallsignalen überwacht. Diese enthalten Informationen über die Blutfluss-Geschwindigkeit. Bei der Komprimierung eines Blutgefässes, insbesondere einer Arterie, beschleunigt sich typischerweise zunächst der Fluss, was zu einer Erhöhung des Dopplersignals führt. Bei kompletter Okklusion verschwindet der Blutfluss und damit das Dopplersignal. Dieser Vorgang kann manuell oder automatisch überwacht werden, um den Okklusionsdruck zu bestimmen. Beispielsweise kann die Okklusion automatisch bestimmt werden, indem der Anpressdruck beim Maximum der Dopplerfrequenz ermittelt wird. Da die Strömungsgeschwindigkeit und damit die Dopplerfrequenz kurz vor der Okklusion am höchsten sind, entspricht der ermittelte Anpressdruck weitgehend dem Okklusionsdruck. Es ist grundsätzlich auch möglich, eine Extrapolation vorzunehmen, um aus dem ermittelten Anpressdruck bei maximaler Dopplerfrequenz den Okklusionsdruck zu ermitteln. Dabei kann der Frequenzverlauf bei niedrigeren Anpressdrücken mitberücksichtigt werden.

Bei einer Variante wird der Blutfluss zusätzlich oder anstelle der Ultraschallsignale anhand von akustischen Signalen (im hörbaren Bereich) überwacht. Derartige akustische Signale können direkt, mittels eines Mikrofons erfasst werden. Als Mikrofon kann dabei z. B. der Drucksensor zur Messung des auf die I<ontal<tfläche wirkenden Anpressdrucks dienen. Die akustischen Signale können aber auch aus dem Dopplersignal, insbesondere aus deren Hüllkurve gewonnen werden, wobei die entsprechenden Signale bei Bedarf frequenzmässig verschoben werden können, um die Erfassbarkeit durch die Bedienperson zu verbessern.

Die akustischen Signale können der Bedienperson über einen Lautsprecher oder Kopfhörer zugänglich gemacht werden.

Generell zeigt eine höhere Schallfrequenz einen schnelleren Blutfluss an. Bei eintretender Okklusion verschwindet der Schall abrupt, womit sich dieses Signal bei einer geeigneten Auswertung dazu eignet, die Okklusion zu detektieren. Bestimmt man beim Verschwinden des Schalls den Druck, entspricht dieser dem Okklusionsdruck.

Bei direkter Schallaufnahme funktioniert das Mikrofon (bzw. der Drucksensor) wie bei einem Stethosl<op und überträgt die Pulsation der Arterie in den Schallraum. Beim Okklusionspunkt wird die Pulsation im Rhythmus des Herzschlags verschwinden, was unmittelbar hörbar ist. Auch auf diese Weise kann somit der Okklusionsdruck ermittelt werden.

Bei einer Gruppe von Ausführungsformen des erfindungsgemässen Verfahrens wird eine Ausdehnung des Blutgefässes anhand von Ultraschallsignalen der Ultraschallsonde bestimmt, wobei die Okklusion einer verschwindenden Ausdehnung in Richtung einer Anpresskraft der Ultraschallsonde entspricht.

Im Einzelstrahlbetrieb (A Mode) werden die Reflexionen des ausgesandten Ultraschallsignals an den Gefässwänden empfangen. Die Distanz zwischen den beiden Reflexionen an der oberen und unteren Gefässwand ergibt sich über die Schallgeschwindigkeit aus dem Laufzeitunterschied. Wird nun mit Hilfe eines von aussen einwirkenden Drucks das Blutgefäss okkludiert, so nähern sich die Reflexionssignale einander an, bis sie nicht mehr unterscheidbar sind. Dieser Punkt entspricht der Okklusion und der entsprechende äussere Druck dem Okklusionsdruck.

Statt im Einzelstrahlbetrieb kann die Ausdehnung des Blutgefässes auch anhand einer Bildgebung mittels Ultraschall-Array ermittelt werden.

Insbesondere bei älteren Personen und bei Patienten mit Diabetes wird eine zunehmende Arteriosklerose festgestellt. Dabei verhärten sich die arteriellen Gefässe aufgrund von Ablagerungen an den Gefässwänden und ihre Elastizität nimmt ab. Will man bei diesen Patienten den Blutdruck mit einer klassischen Manschette messen, führt die Arteriosklerose zu einem zu hohen und damit falschen oder zumindest unpräzisen Messwert des Blutdrucks.

Bei einer weiteren Gruppe von Ausführungsformen wird deshalb aus mindestens zwei Wertepaaren von Anpressdruck und Ausdehnung des Blutgefässes in Richtung der Anpresskraft ein Elastizitätswert des Blutgefässes bestimmt, und der Elastizitätswert wird bei der Bestimmung des zu messenden Innendrucks berücksichtigt. Er kann beispielsweise als Korrekturfaktor in die Bestimmung des Blutdrucks anhand des Okklusionsdrucks einfliessen.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1A, 1B: eine Seitenansicht und ein Schrägbild eines ersten Ausführungsbeispiels einer erfindungsgemässen Ultraschallsondenanordnung;
- Fig. 2: einen Querschnitt durch den Aufsatz des ersten Ausführungsbeispiels;
- Fig. 3: einen Querschnitt durch einen auf die Ultraschallsonde aufgesetzten Aufsatz;
- Fig. 4A, 4B: eine schematische Darstellung eines Messablaufs bei minimalem Anpressdruck und bei Okklusionsdruck; und
- Fig. 5: eine Frontansicht eines zweiten Ausführungsbeispiels einer erfindungsgemässen Ultraschallsondenanordnung.

Grundsätzlich sind in den Figuren gleiche Teile mit gleichen Bezugszeichen versehen.

### Wege zur Ausführung der Erfindung

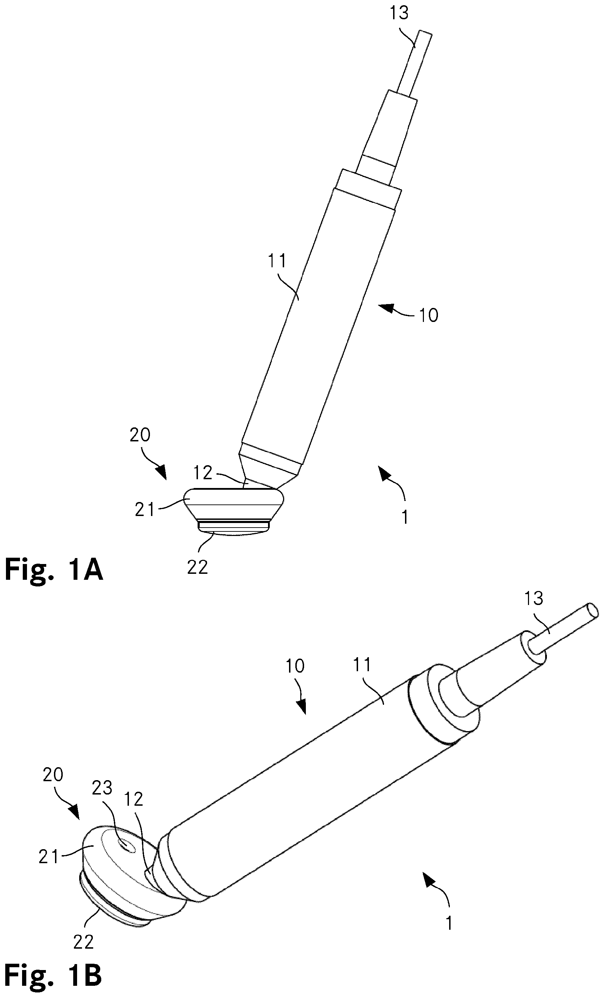

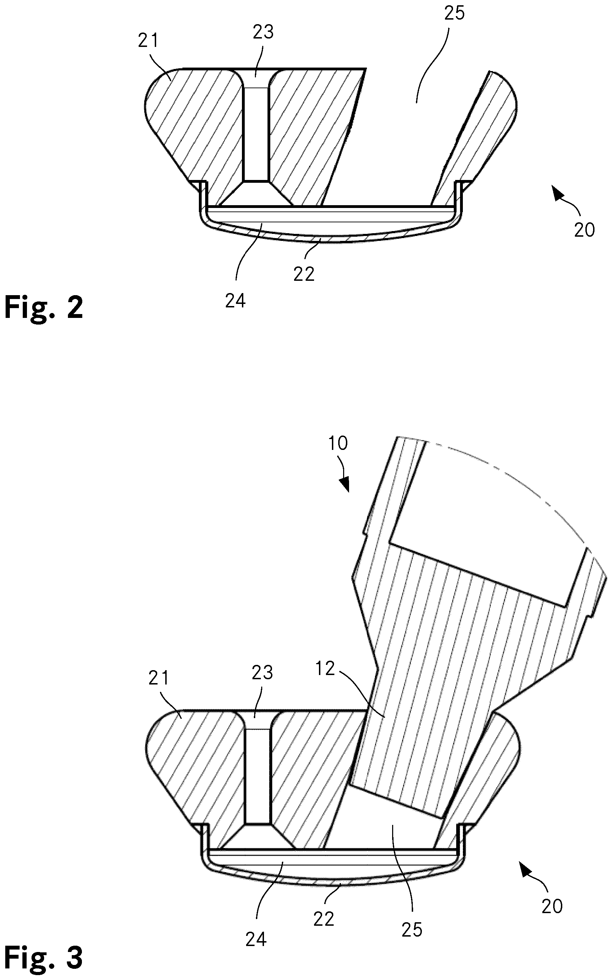

Die Figuren 1A, 1B sind eine Seitenansicht und ein Schrägbild eines ersten Ausführungsbeispiels einer erfindungsgemässen Ultraschallsondenanordnung. Die Figur 2 zeigt einen Querschnitt durch den Aufsatz des ersten Ausführungsbeispiels. Die Figur 3 zeigt einen Querschnitt durch einen auf die Ultraschallsonde aufgesetzten Aufsatz.

Die Ultraschallsondenanordnung 1 umfasst eine Ultraschallsonde 10 und einen Aufsatz 20. Die Ultraschallsonde 10 umfasst einen im Wesentlichen zylindrischen Grundkörper 11 und einen distalen Abschnitt 12, der eine konische Geometrie mit kreisförmigem Querschnitt aufweist und im Vergleich zum Grundkörper 11 verjüngt ist. Über ein Kabel 13 ist die Ultraschallsonde mit einem Auswertegerät verbunden. Die Ultraschallsonde 10 weist in an sich bekannter Weise einen Ultraschall-Transducer auf und ist zur Vornahme von Einzelstrahl-Doppler-Ultraschall-Messungen ausgebildet.

Entsprechende Ultraschallsonden sind kommerziell erhältlich, z. B. von der Huntleigh Healthcare Ltd, Cardiff (UI<).

Der Aufsatz 20 umfasst einen Grundkörper 21 auf dessen distaler Seite eine Membran 22 aus Silikonkautschuk mit einer Härte von 40 Shore A angeordnet ist. Der Grundkörper 21 und die Membran 22 haben jeweils einen kreisförmigen Querschnitt, wobei sich der Grundkörper 21 in distaler Richtung verjüngt und auf der distalen Seite einen Haltering ausbildet.

Die Membran 22 und der Grundkörper 21 begrenzen einen Aufnahmeraum 24 für eine ultraschalltransparente Flüssigkeit, z. B. ein Öl mit einer Viskosität in einer Viskositätsklasse von 32 - 68 ISO VG (nach DIN ISO 3448:2010). Beim Öl kann es sich insbesondere um ein synthetisches, mineralisches und/oder pflanzliches Öl handeln. Ein Kanal 23 mündet von der Rückseite des Aufsatzes 20 bis in den Aufnahmeraum 24.

Der distale Abschnitt 12 der Ultraschallsonde 10 ist von einer Sondenaufnahme 25 des Aufsatzes 20 mit konischer Innengeometrie aufgenommen. Die Aufnahme erstreckt sich von der proximalen Seite des Aufsatzes 20 bis zum Aufnahmeraum 24, wobei die Öffnung auf der proximalen Seite weiter von der Symmetrieachse des Aufsatzes 20 entfernt ist als deren distales Ende bei der proximalen Begrenzung des Aufnahmeraums 24. Die Symmetrieachse der Sondenaufnahme 25 (und damit des distalen Abschnitts 12 der aufgenommenen Ultraschallsonde 10) weist relativ zur Hauptebene, die durch die Membran 22 definiert wird (und senkrecht zur Symmetrieachse des Aufsatzes 20 verläuft), einen Winkel von ca. 70° auf.

Ein Drucksensor, z. B. ein piezoresistiver Sensor, kann unmittelbar in den Kanal 23 eingesetzt oder über eine Schlauchleitung mit diesem verbunden werden, um den Druck im Aufnahmeraum 24 und damit den Anpressdruck der Ultraschallsondenanordnung 1 auf die untersuchte I<örperoberfläche zu messen.

Die Anzeige der Messwerte des Ultraschallsensors bzw. des Drucksensors oder davon abgeleiteter Informationen erfolgt insbesondere gemeinsam auf einem Display. So können der Anpressdruck und seine Auswirkungen auf den Blutfluss bzw. die Geometrie eines untersuchten Blutgefässes nebeneinander angezeigt und mit Vorteil auch gemeinsam verarbeitet werden. Dazu sind der Drucksensor und der Ultraschallsensor mit einer Verarbeitungseinheit verbunden, die wiederum das Display ansteuert.

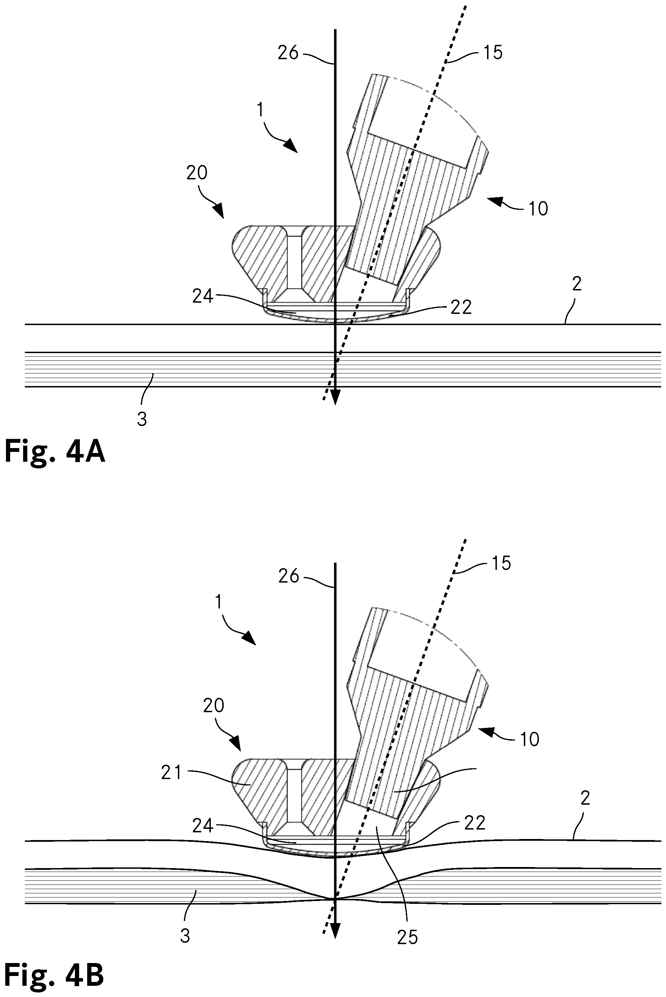

Die Figuren 4A, 4B sind eine schematische Darstellung eines Messablaufs bei minimalem Anpressdruck bzw. bei Okklusionsdruck. In der Figur 4A ist dargestellt, wie mit minimalem Anpressdruck eine erste Messung durchgeführt wird. Die Membran 22 kontaktiert mit ihrer äusseren Fläche (I<ontal<tfläche) die Hautoberfläche 2 des Patienten. Die Ultraschallsondenanordnung 1 ist so positioniert und orientiert, dass die Mittelachse der Ultraschallsonde 10, welche dem Messstrahl 15 entspricht, oberhalb eines Blutgefässes 3, insbesondere einer Arterie, zu liegen kommt. Die durch die Membran 22 definierte Hauptfläche ist parallel zur Hautoberfläche 2 am Kontaktpunkt. Die Wirkachse 26, entsprechend der Mittelachse des Aufsatzes 20, steht also senkrecht auf der Hautoberfläche 2. Diese Wirkachse 26 und der Messstrahl 15 schneiden sich im Bereich des Blutgefässes 3, wobei hier eine gewisse Abweichung unkritisch ist.

Beim minimalen Anpressdruck liegt keine oder nur eine unwesentliche Kompression des Blutgefässes 3 vor.

Der Anpressdruck wird nun ausgehend von der Situation in der Figur 4A erhöht, indem vom Nutzer entlang der Wirkachse 26 eine Anpresskraft auf die Ultraschallsondenanordnung ausgeübt wird. Der Anpressdruck wird durch die Druckmessung der Flüssigkeit im Aufnahmeraum 24 überwacht. Gleichzeitig wird mittels der Ultraschallsonde 10 der Blutfluss im Blutgefäss 3 überwacht. Mittels Dopplermessungen kann die Flussgeschwindigkeit ermittelt werden. Ergänzend kann aus dem Dopplersignal ein akustisches Signal erzeugt und über einen Lautsprecher oder Kopfhörer ausgegeben werden. Anhand dieses akustischen Signals kann der Nutzer intuitiv erkennen, ob im Blutgefäss 3 ein Blutfluss stattfindet oder nicht.

Der Anpressdruck wird nun so lange erhöht, bis der Blutfluss im Blutgefäss 3 aufgrund von dessen Kompression unterbunden ist, also eine Okklusion vorliegt. Der entsprechende Anpressdruck ergibt sich aus der Druckmessung im Aufnahmeraum 24.

Wird ein geeignetes Blutgefäss untersucht, insbesondere ein oberflächliches arterielles Blutgefäss, das sich vor einem vergleichsweise harten Gewebe (z. B. einem Knochen oder einer Faszie) befindet, kann nun aus dem Okklusionsdruck unmittelbar der (systolische) Blutdruck bestimmt werden. Ein geeignetes Blutgefäss ist z. B. die Arteria Brachialis, die überdies den Vorteil bietet, dass in verschiedenen I<örperstellungen eine Messung ungefähr auf Herzhöhe möglich ist, so dass im Gegensatz zu Messungen an anderen Körperteilen keine Korrektur für den statischen Druck erforderlich ist. Die erfindungsgemässe Vorrichtung ist auch beispielsweise zur Blutdruckmessung an Arterien im Fussbereich geeignet, um z. B. die Qualität der Blutversorgung der unteren Extremitäten zu beurteilen, beispielsweise bei diabetischen Patienten.

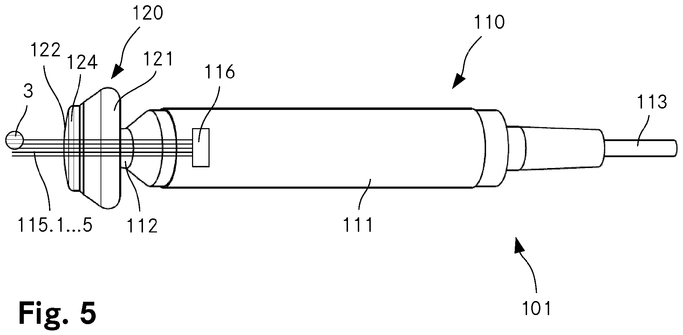

Die Figur 5 ist eine Frontansicht eines zweiten Ausführungsbeispiels einer erfindungsgemässen Ultraschallsondenanordnung. Die Ultraschallsondenanordnung 101 entspricht in vielerlei Hinsicht derjenigen des ersten Ausführungsbeispiels. So umfasst sie eine Ultraschallsonde 110 und einen Aufsatz 120. Die Ultraschallsonde 110 umfasst einen im Wesentlichen zylindrischen Grundkörper 111 und einen distalen Abschnitt 112, der eine konische Geometrie mit kreisförmigem Querschnitt aufweist und im Vergleich zum Grundkörper 111 verjüngt ist. Über ein Kabel 113 ist die Ultraschallsonde 110 mit einem Auswertegerät verbunden. Die Ultraschallsonde 110 weist nun aber ein Array 116 mit fünf in Reihe angeordneten Ultraschall-Transducern auf, die fünf parallele Messstrahlen 115.1 ... 5 erzeugen.

Der Aufsatz 120 umfasst einen Grundkörper 121 auf dessen distaler Seite eine Membran 122 aus Silikonkautschuk mit einer Härte von 40 Shore A angeordnet ist. Der Grundkörper 121 und die Membran 122 haben jeweils einen kreisförmigen Querschnitt, wobei sich der Grundkörper 121 in distaler Richtung verjüngt und auf der distalen Seite einen Haltering ausbildet.

Die Membran 122 und der Grundkörper 121 begrenzen wiederum einen Aufnahmeraum 124 für eine ultraschalltransparente Flüssigkeit. Ein Kanal mündet von der Rückseite des Aufsatzes 120 bis in den Aufnahmeraum 124.

Der distale Abschnitt 112 der Ultraschallsonde 110 ist von einer Aufnahme des Aufsatzes 120 mit konischer Innengeometrie aufgenommen. Die Aufnahme erstreckt sich von der proximalen Seite des Aufsatzes 120 bis zum Aufnahmeraum 124, wobei die Öffnung auf der proximalen Seite weiter von der Symmetrieachse des Aufsatzes 120 entfernt ist als deren distales Ende bei der proximalen Begrenzung des Aufnahmeraums 124. Die Symmetrieachse der Aufnahme (und damit des distalen Abschnitts 112 der aufgenommenen Ultraschallsonde 110) weist relativ zur Hauptebene, die durch die Membran 122 definiert wird (und senkrecht zur Symmetrieachse des Aufsatzes 120 verläuft), einen Winkel von ca. 70° auf.

Ein Drucksensor, z. B. ein piezoresistiver Sensor, kann unmittelbar in den Kanal eingesetzt oder über eine Schlauchleitung mit diesem verbunden werden, um den Druck im Aufnahmeraum 124 und damit den Anpressdruck der Ultraschallsondenanordnung 101 auf die untersuchte I<örperoberfläche zu messen.

Die fünf Messstrahlen 115.1 ... 5 ermöglichen nun eine Erfassung des Blutgefässes 3 auch dann, wenn die Sonde seitlich versetzt platziert ist. Es ist möglich, eine seitliche Abweichung zwischen Ultraschallsondenanordnung 101 und Blutgefäss 3 zu erkennen und beim Erkennen der Okklusion zu berücksichtigen. Die Messung kann entsprechend korrigiert werden und/oder der Nutzer wird aufgefordert, die Sondenanordnung wieder zu zentrieren.

Insbesondere bei älteren Menschen reduziert sich die Elastizität der Blutgefässe, die sog. Compliance, aufgrund von Arteriosklerose. Dies führt zu einer Erhöhung des gemessenen systolischen Drucks, weil der für die Okklusion notwendige äussere Anpressdruck vergrössert wird.

Mit Hilfe einer erfindungsgemässen Ultraschallsondenanordnung gemäss der ersten oder zweiten Ausführungsform lässt sich ein Elastizitätswert des Blutgefässes ermitteln und bei der Bestimmung des Blutdrucks berücksichtigen. Grundsätzlich wird dabei die Kompression des Blutgefässes anhand eines geometrischen Masses, das die Ausdehnung in Einwirl<richtung der Anpresskraft repräsentiert, bei mindestens zwei verschiedenen Werten des Anpressdrucks ermittelt. Daraus lässt sich dann ein Mass für die Elastizität bestimmen.

In einer Ausführungsform eines entsprechenden Verfahrens wird zunächst über die mittels der Ultraschallsonde erfassten Reflexionssignale ein erster Durchmesser D₀ des Blutgefässes bei minimalem Anpressdruck bestimmt. Danach wird die Anpresskraft erhöht, bis der Durchmesser um einen vorgegebenen Prozentsatz, z. B. 10%, verringert ist. Der entsprechende Durchmesser D₁ und der dazugehörige Druckwert p₁ werden gespeichert.

Als nächstes wird die Anpresskraft weiter erhöht, bis der Durchmesser gegenüber dem Durchmesser D₁ um ein vorgegebenes Mass, z. B. 70% verringert ist. Der entsprechende Druckwert p₂ und der Durchmesser D₂ werden wiederum gespeichert.

Es ist zu beachten, dass aufgrund der angewinkelten Orientierung der Ultraschallsonde die Ultraschallechos einen Wert für den Durchmesser anzeigen, der grösser ist als der tatsächliche Wert. Dies kann grundsätzlich anhand des bekannten Winkels ohne Weiteres kompensiert werden. Im Rahmen der dargestellten Ausführungsbeispiele ist dies aber an sich nicht notwendig, weil nur relative Grössen bzw. Verhältnisse verwendet werden.

Nun erfolgt die Berechnung eines Quotienten Q = p₂/p₁. Dieser Quotient kann mit dem Normwert gesunder Personen verglichen werden und ermöglicht eine quantitative Aussage über den Grad der Versteifung des Blutgefässes. Ein hoher Wert von Q deutet auf einen erhöhten Kompressionsdruck und damit auf ein versteiftes Blutgefäss hin. Q oder eine davon abgeleitete Grösse ist als Korrekturfaktor geeignet, um den mit dem gleichen System im gleichen Messprozess gemessen Okklusionsdruck zu korrigieren. Damit wird ist es möglich, unabhängig von der Elastizität der Gefässe den wahren Druck im Blut bzw. den Blutdruck zu bestimmen.

In einer alternativen Ausführungsform dieses Verfahrens wird ein die Elastizität repräsentierender Quotient bestimmt, indem Druckwerte vorgegeben werden und die entsprechenden Durchmesser in ein Verhältnis gesetzt werden.

Die Erfindung ist nicht auf die dargestellten Ausführungsbeispiele beschränkt. So können insbesondere die Geometrie und/oder Materialisierung des Aufsatzes anders ausgeführt sein. In einer ersten Variante ist beispielsweise der Aufsatz einstückig aus einem einheitlichen Material ausgeführt, wobei unterschiedliche Härten durch unterschiedliche Materialdicken erreicht werden. In einer weiteren Variante werden z. B. Silikonmaterialien unterschiedlicher Härte verwendet. Der Messprozess und die Auswertung der Messdaten können mehr oder weniger automatisiert ablaufen.

Zusammenfassend ist festzustellen, dass die Erfindung einen Aufsatz für eine Ultraschallsonde schafft, welcher eine einfache und zuverlässige Blutdruckmessung ermöglicht.

## Patentansprüche

1. Aufsatz für eine Ultraschallsonde, umfassend
a) einen Grundkörper mit einer Aufnahme für einen distalen Endbereich der Ultraschallsonde;
b) eine distal am Grundkörper angeordnete I<ontal<tfläche, die durch eine ultraschalltransparente Membran gebildet ist;
c) einen Drucksensor zur Messung eines auf die I<ontal<tfläche wirkenden Anpressdrucks;
wobei
d) die Aufnahme für die Ultraschallsonde und die I<ontal<tfläche derart angeordnet sind, dass ein Messstrahl der Ultraschallsonde die ultraschalltransparente Membran durchdringt und dass zwischen dem Messstrahl und einer Hauptfläche der I<ontal<tfläche ein Winkel von 35-80°, insbesondere 45-75°, gebildet ist.

2. Aufsatz nach Anspruch 1, **dadurch gekennzeichnet, dass** zwischen der ultraschalltransparenten Membran und dem Grundkörper ein Aufnahmeraum für eine ultraschalltransparente Flüssigkeit ausgebildet ist und dass der Drucksensor zur Messung eines Drucks in der Flüssigkeit ausgebildet ist.

3. Aufsatz nach Anspruch 1 oder 2, **dadurch gekennzeichnet, dass** die ultraschalltransparente Membran eine kreisförmige Grundfläche hat und dass die Aufnahme derart aussermittig am Grundkörper angeordnet ist, dass der Messstrahl die ultraschalltransparente Membran in einem zentralen Bereich durchdringt, der einer Kreisfläche mit einem Aussenradius vom halben Aussenradius der kreisförmigen Grundfläche entspricht.

4. Aufsatz nach einem der Ansprüche 1 bis 3, **dadurch gekennzeichnet, dass** die Aufnahme konisch ausgebildet ist.

5. Ultraschallsondenanordnung, umfassend eine Ultraschallsonde und einen Aufsatz nach einem der Ansprüche 1 bis 4.

6. Ultraschallsondenanordnung nach Anspruch 5, **dadurch gekennzeichnet, dass** es sich bei der Ultraschallsonde um eine Einzelstrahl-Ultraschallsonde handelt.

7. Ultraschallsondenanordnung nach Anspruch 5, **dadurch gekennzeichnet, dass** die Ultraschallsonde zum Generieren mehrerer Messstrahlen entlang einer Linie ausgebildet ist.

8. Verfahren zur Messung eines Innendrucks in einem Blutgefäss mit einer Ultraschallsondenanordnung, insbesondere mit einer Ultraschallsondenanordnung nach einem der Ansprüche 5 bis 7, umfassend folgende Schritte:
a) Aufsetzen der Ultraschallsondenanordnung auf die I<örperoberfläche eines Patienten, an einem Aufsetzort;
b) Komprimieren eines Blutgefässes unterhalb des Aufsetzorts durch Erhöhung des Anpressdrucks einer Kontaktfläche der Ultraschallsondenanordnung auf die I<örperoberfläche;
c) Erkennen einer Okklusion des Blutgefässes anhand von Ultraschallsignalen einer Ultraschallsonde der Ultraschallsondenanordnung, wobei der zu messende Innendruck aus dem mittels eines Drucksensors der Ultraschallsondenanordnung gemessenen Anpressdruck der Ultraschallsondenanordnung bei einsetzender Okklusion bestimmt wird.

9. Verfahren nach Anspruch 8, **dadurch gekennzeichnet, dass** ein Blutfluss im Blutgefäss anhand von Ultraschallsignalen der Ultraschallsonde überwacht wird, wobei die Okklusion anhand eines ausbleibenden Blutflusses erkannt wird.

10. Verfahren nach Anspruch 9, **dadurch gekennzeichnet, dass** der Blutfluss anhand von Doppler-Ultraschallsignalen überwacht wird.

11. Verfahren nach Anspruch 9 oder 10, **dadurch gekennzeichnet, dass** der Blutfluss anhand von akustischen Signalen überwacht wird.

12. Verfahren nach einem der Ansprüche 8 bis 11, **dadurch gekennzeichnet, dass** eine Ausdehnung des Blutgefässes anhand von Ultraschallsignalen der Ultraschallsonde bestimmt wird, wobei die Okklusion einer verschwindenden Ausdehnung in Richtung einer Anpresskraft der Ultraschallsonde entspricht.

13. Verfahren nach Anspruch 12, **dadurch gekennzeichnet, dass** aus mindestens zwei Wertepaaren von Anpressdruck und Ausdehnung des Blutgefässes in Richtung der Anpresskraft ein Elastizitätswert des Blutgefässes bestimmt wird und dass der Elastizitätswert bei der Bestimmung des zu messenden Innendrucks berücksichtigt wird.

14. Verfahren nach einem der Ansprüche 8 bis 13, **dadurch gekennzeichnet, dass** eine Ultraschallsondenanordnung nach Anspruch 7 verwendet wird, wobei anhand der mehreren Messstrahlen eine seitliche Abweichung zwischen Ultraschallsonde und Blutgefäss erkannt und beim Erkennen der Okklusion berücksichtigt wird.
